# EUROPEAN PATENT APPLICATION

(11) **EP 4 258 276 A1**
(43) Date of publication of application: **11.10.2023**
(21) Application number: 23157873.3
(22) Date of filing: 21.02.2023
(51) Int. Cl.: G16H 20/30, G16H 40/63, A63B 21/00, A63B 24/00, H04W 4/38

(54) **TRANSFER OF EXERCISE DATA FROM AN EXERCISE DEVICE**

(30) Priority: 04.04.2022 SE 2250429
(71) Applicant: Sony Group Corporation, Tokyo 108-0075 (JP)
(72) Inventor: BENGTSSON, Henrik, Basingstoke, RG22 4SB (GB); HALLQVIST, Magnus, Basingstoke, RG22 4SB (GB); FREDLUND, Anders, Basingstoke, RG22 4SB (GB); BJÖRNESKOG, Peter, Basingstoke, RG22 4SB (GB); BOROWSKI, Jimmy, Basingstoke, RG22 4SB (GB); RYDBERG, Jens, Basingstoke, RG22 4SB (GB); AFRIDI, Khalid, Basingstoke, RG22 4SB (GB)
(74) Representative: Neij & Lindberg AB

(57) **Abstract**

A monitoring arrangement (20) for an exercise device (10) generates data items representing exercise activity by a user, performs a connection process for establishment of an end-to-end connection to a user device (30), broadcasts progress data that represents data items generated during the connection process, and sequentially transmits, on the end-to-end connection data items, generated after establishment of the end-to-end connection. The user device (30) receives the progress data and presents, during the connection process, progress of the exercise activity based on the progress data. After establishment of the end-to-end connection, the user device (30) presents progress of the exercise activity based on data items received on the end-to-end connection. The combination of broadcasting and sequential transmission obviates the need for the user to wait for the end-to-end connection before starting exercise activity.

## Description

### Technical Field

The present disclosure relates generally to exercise devices, and, more particularly, to techniques for providing exercise feedback to a user of an exercise device.

### Background

Exercise and physical fitness are steadily gaining in popularity. The growing interest in physical fitness is reflected by the growing number of gyms found in both public and private settings.

Exercise machines are often used for physical exercise, for example weight machines in which stacked weight plates are lifted by the user against the action of gravity. Conventionally, the user has to keep a manual record of the exercises performed on the machines and the outcome of the respective exercise.

Recently, automated systems have been developed to help a user to track and record progress on exercise machines. Such automated systems comprise a local detector on the respective exercise machine. The local detector is configured to determine exercise data that represents an exercise activity performed by a user of the exercise machine and transmit the exercise data to a local or remote receiving device. The exercise data may be transmitted in real-time, enabling the system to provide workout feedback in real time to the user, for example on a display of the local receiving device. An example of such a system is described in WO2018/162763.

One challenge in this context is to ensure that exercise data is made available at the local receiving device during the exercise activity so that the workout feedback is provided to the user throughout the exercise activity. The local detector is typically battery-powered, so power consumption is another concern. It is also desirable for the cost and complexity of the local detector to be low.

### Summary

It is an objective to at least partly overcome one or more limitations of the prior art.

Another objective is to provide an improved technique of transferring exercise data from an exercise device to a user device.

A further objective is to provide such a technique that enables detailed feedback to be provided to the user about the progress of individual repetitions performed by the user of the exercise device, while ensuring that feedback is available throughout the exercise activity.

One or more of these objectives, as well as further objectives that may appear from the description below, are at least partly achieved by a monitoring arrangement and an electronic device, embodiments thereof being defined by the dependent claims.

According to various aspects of the present disclosure, a monitoring arrangement for installation in an exercise device is operable to perform a connection process to establish an end-to-end connection to a user device. When the end-to-end connection is established, the monitoring arrangement is operable to sequentially transmit data items, which represent an exercise activity performed by a user of the exercise activity, to a user device. The monitoring arrangement is thus operable to "stream" the data items on the end-to-end connection. The user device is thereby enabled to provide feedback, for example in real time, about the progress of the exercise activity to the user. It takes time for the connection process to establish the end-to-end connection, and the duration of the connection process may vary from time to time. The connection process may even fail. According to various aspects of the present disclosure, the monitoring arrangement is further operable to broadcast exercise-related data ("progress data") during the connection progress, for receipt by the user device, which is thereby enabled to provide feedback about the progress of the exercise activity to the user also during the connection process. The combination of broadcasting and streaming obviates the need for the user to wait for the end-to-end connection to be established before starting the exercise activity. Conversely, the combination enables the user device to provide feedback to the user throughout the exercise activity, should the user start the exercise activity during the connection process.

Still other objectives and aspects, as well as features, embodiments and advantages will appear from the following detailed description, from the attached claims as well as from the drawings.

### Brief Description of Drawings

FIGS 1A-1C depict an example exercise machine with a position sensor for use in monitoring of exercise activity, and FIG. 1D depicts an example handheld exercise device with integrated motion sensor.
FIG. 2 shows an example of data communication in an exercise system.
FIGS 3-4 are block diagrams of an example monitoring arrangement and an example user device, respectively, in an exercise system.
FIGS 5A-5B are flow charts of example methods of operating a monitoring arrangement and a user device, respectively, for monitoring of exercise activity.
FIG. 6A is a sequence diagram of an example startup procedure in the exercise system of FIG. 2, and FIGS 6B-6E illustrate procedures performed by a monitoring arrangement in accordance with various examples.
FIG. 7 is a flowchart of an example feedback procedure performed by a user device.
FIGS 8A-8C show examples of images displayed on a user device during the feedback procedure of FIG. 7.
FIG. 9 is a flowchart of an example calibration procedure performed by a user device during exercise activity.

### Detailed Description of Example Embodiments

Embodiments will now be described more fully hereinafter with reference to the accompanying drawings, in which some, but not all, embodiments are shown. Indeed, the subject of the present disclosure may be embodied in many different forms and should not be construed as limited to the embodiments set forth herein; rather, these embodiments are provided so that this disclosure may satisfy applicable legal requirements.

Also, it will be understood that, where possible, any of the advantages, features, functions, devices, and/or operational aspects of any of the embodiments described and/or contemplated herein may be included in any of the other embodiments described and/or contemplated herein, and/or vice versa. In addition, where possible, any terms expressed in the singular form herein are meant to also include the plural form and/or vice versa, unless explicitly stated otherwise. As used herein, "at least one" shall mean "one or more" and these phrases are intended to be interchangeable. Accordingly, the terms "a" and/or "an" shall mean "at least one" or "one or more", even though the phrase "one or more" or "at least one" is also used herein. As used herein, except where the context requires otherwise owing to express language or necessary implication, the word "comprise" or variations such as "comprises" or "comprising" is used in an inclusive sense, that is, to specify the presence of the stated features but not to preclude the presence or addition of further features in various embodiments.

As used herein, the terms "multiple", "plural" and "plurality" are intended to imply provision of two or more elements. The term "and/or" includes any and all combinations of one or more of the associated listed elements.

It will furthermore be understood that, although the terms first, second, etc. may be used herein to describe various elements, these elements should not be limited by these terms. These terms are only used to distinguish one element from another. For example, a first element could be termed a second element, and, similarly, a second element could be termed a first element, without departing the scope of the present disclosure.

Well-known functions or constructions may not be described in detail for brevity and/or clarity. Unless otherwise defined, all terms (including technical and scientific terms) used herein have the same meaning as commonly understood by one of ordinary skill in the art to which this disclosure belongs.

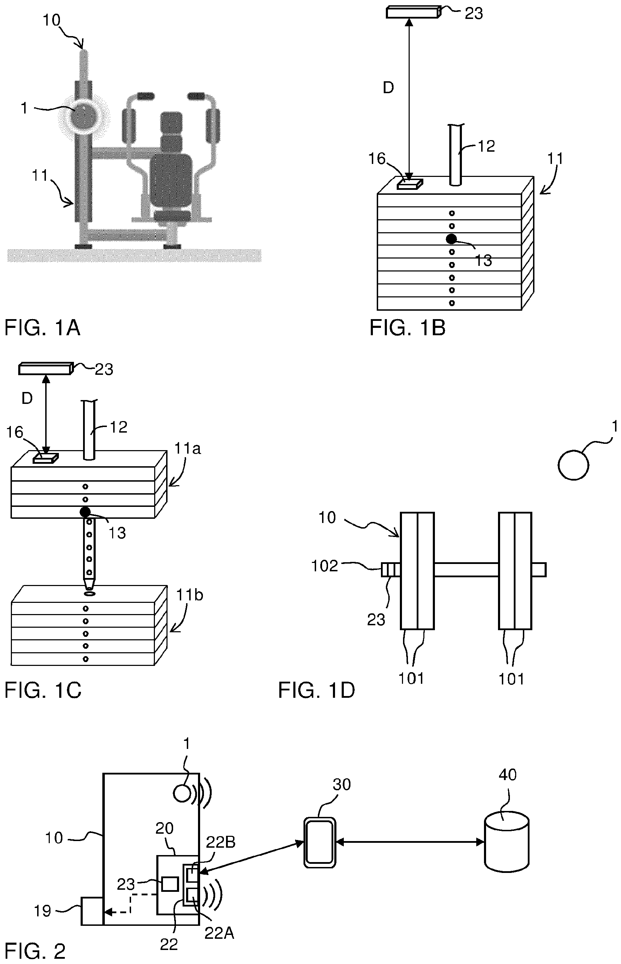

FIG. 1A is an isometric view of a stacked weight exercise machine 10 having a plurality of weights 11. FIGS 1B-1C depict the arrangement of weights 11 in further detail. The exercise machine 10 comprises a lifting mechanism 12 and an engaging member ("selector pin") 13 for selectively engaging a number of stacked weights to the lifting mechanism 12. The lifting mechanism 12 may be coupled, in a manner well known to the skilled person, to one or more gripping or pushing members via one or more cables, belts, rods, etc. In the illustrated example, the lifting mechanism 12 includes a support member having a rod-shaped portion, configured to pass vertically through corresponding holes in the weights 11. The support member 12 may further include a top portion, such as a fixed top weight. A sensor 23 is arranged in the exercise machine 10 to measure the movement of the weights 11. In the illustrated example, the sensor 23 is a time-of-flight (ToF) sensor, which is arranged on top of the stacked weights 11 and is thus moving when a user performs an exercise in the machine 10. A reflective element ("reflector") 16 is located at a fixed position spaced from the top of the weights 11. For example, the reflector 16 may be attached to or part of the frame of the machine 10. In a variant, not shown, the positions of the ToF sensor 23 and the reflector 16 may be reversed. Time-of-flight (ToF) is an established technique for distance determination ("range finding" or "ranging") and involves measuring the roundtrip time of a signal provided by a source (sensor 23) onto a target (reflector 16), with the distance D being given by half the roundtrip time multiplied by the propagation speed of the signal. The signal may be an ultrasound signal or an electromagnetic signal. In some embodiments, the signal is a light signal in the ultraviolet, visible or infrared wavelength range. The light signal may be generated by a laser or an LED.

In exercise machines comprising stacked weights, the user may typically select how many of the weights should be used or engaged in the exercise, by inserting the selector pin 13 in one of the weights. During exercise, the user will then lift the selected weights, as exemplified in FIG. 1C, where the selected portion 11a of the stacked weights is lifted in relation to a remaining portion 11b. The training may be tracked by monitoring how many times the selected weights are lifted (number of repetitions) and, optionally, how many weights are selected to be lifted.

The exercise machine 10 has a "rest state", which is attained when the user does not apply force to the machine 10. In the example of FIGS 1B-1C, the rest state (FIG. 1B) results in a maximum distance ("rest distance") between the ToF sensor 23 and the reflector 16. When not in the rest state, the machine 10 is in a loaded state (FIG. 1C) at which the distance is smaller than the maximum distance. The exercise machine may or may not attain the rest state between repetitions, depending on the training schedule of the specific user.

The sensor 23 is configured to provide sensor data indicative of measured distance D between the sensor 23 and the reflector 16. The sensor data thereby enables determination of the number of repetitions. Although not shown, one or more further sensors (not shown) may be provided to provide further sensor data, for example indicative of the selected weights. For example, as disclosed in WO2015/113162 and WO2017/178048, such a further sensor may be arranged to measure the distance between the selector pin 13 and a reference point, which is located such that the measured distance is indicative of the selected weight. The further sensor may also be a ToF sensor, and the distance may be measured between the selector pin 13 and a reference point on the selected portion 11a above the selector pin 13 (for example, on top of the stacked weights) or between the selector pin 13 and a stationary reference point beneath the selector pin 13.

FIGS 1A-1C are given to provide a non-limiting example of an exercise machine 10. An exercise machine is any type of stationary device that allows a user to perform an exercise activity that comprises repetitions of a predefined movement of a moveable element of the exercise machine (cf. stacked weights 11 in FIGS 1B-1C). The predefined movement may be constrained by the exercise machine. For example, the exercise machine may be configured to mechanically limit the movement of the moveable element. In other exercise machines, such as cable machines, the movement is less restricted. Nevertheless, at least one predefined movement is defined for each exercise machine. Conventionally, performance of one predefined movement is denoted a "repetition", and a sequence of repetitions is denoted a "set". Often, a repetition comprises an eccentric phase and a concentric phase, in any order. Typically, a set is performed without pause between the repetitions, whereas the user pauses between sets if more than one set is performed. A repetition and/or a set may be seen as a "sub-target" to be achieved during the exercise activity.

The term "exercise device", as used herein, includes not only exercise machines but also so-called free weights. Free weights include dumbbells, barbells, medicine balls, kettlebells, etc. FIG. 1C is a side view of an exercise device 10 in the form of a dumbbell having a number of weight plates 101 mounted on a main rod 102. A sensor 23 is attached to or integrated with the dumbbell 10 to measure the movement of the dumbbell 10 during an exercise activity. In one example, the sensor 23 comprises one or more accelerometers, for example as part of an inertial measurement unit (IMU). Although exercise is unrestricted for free weights and many different exercise activities may be performed with the dumbbell 10, each exercise activity comprises a predefined movement to be performed in repetitions and sets.

The sensors described above are merely examples of a measurement device 23 which is arranged at, in or on the exercise device 10 to generate sensor data representing an exercise activity performed by a user of the exercise device 10. Non-limiting examples of sensors that may be part of the measurement device 23 include position encoders, ranging sensors, accelerometers, triangulation systems, trilateration systems, and computer vision systems. The measurement device 23 may or may not be a unitary component. The measurement device 23 is configured to repeatedly, at a fixed or variable sampling rate, generate data items. Thus, the sensor data is provided as a time sequence or "stream" of data items. The respective data item may comprise raw data or pre-processed data at a respective time point. In the example of a ToF sensor for measuring exercise movement, the respective data item may comprise a round-trip time or a distance (D). In the example of an IMU, the respective data item may comprise accelerometer data or a relative position. The respective data item may also include a time stamp, if a variable sampling rate is used. In the following, it is assumed that the respective data item comprises position data indicative of a momentary position of the exercise device or a moveable element within the exercise device. Thereby, the stream of data items represents a movement trajectory as a function of time, i.e. a movement pattern. Depending on exercise device, the movement trajectory may be one-, two- or three-dimensional.

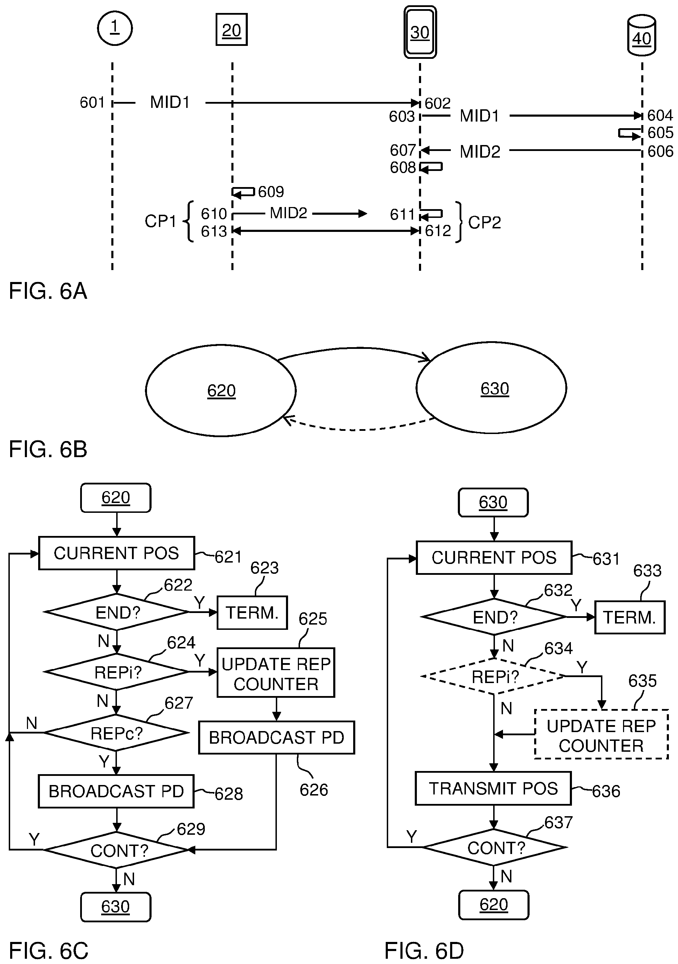

The provision of the measurement device 23 enables the exercise device 10 to be included in an automated system for tracking and recording exercise activity. FIG. 2 shows an example of such a system, which includes a plurality of exercise devices 10 (one shown), a handheld electronic device ("user device") 30 of each user, and a local or remote back-end computer ("server") 40. In the illustrated example, the respective exercise device 10 is associated with an electronic tag 1, which may be attached to the exercise device 10 (as shown in FIG. 1A) or arranged nearby the exercise device 10 (as shown in FIG. 1D). The tag 1 enables a user to associate the user device 30 with the exercise device 10 before initiating an exercise activity on the exercise device 10. The tag 1 is configured to transmit (broadcast) a tag identifier for receipt by the user device 30, which is thereby capable of identifying the exercise device 10, for example by querying the server 40. As shown, the exercise device 10 comprises a monitoring arrangement 20 ("local detector"), which includes the measurement device 23 and a communication device 22 for wireless data transfer to the user device 30. As will be described in more detail below, the communication device 22 is operable to switch between a first communication mode, in which data is broadcast for interception by the user device 30, and a second communication mode, in which data is "streamed" on an end-to-end connection to the user device 30. In the example of FIG. 2, the first mode is represented by module 22A, and the second mode is represented by module 22B. The end-to-end connection is a wireless communication channel which is established and maintained for two-way communication between the communication device 22 and the user device 30. Thus, the second mode may be regarded as an "online mode". By contrast, the broadcasting in the first mode is not made on an end-to-end connection but relies on the user device 30 being operable to autonomously intercept the broadcasts. Thus, the first mode may be regarded as an "offline mode". For example, the broadcasts may not be specifically intended for the user device and may not include an identifier of the user device 30. Instead, the user device 30 may receive and operate on the broadcasts based on an origin identifier included in the broadcasts. The origin identifier may be known to the user device 30 based on the above-mentioned tag identifier. The use of tag and origin identifiers is further described below with reference to FIG. 6A.

The system in FIG. 2 may be configured to store a record of the exercise activity performed by the user, in the user device 30 and/or at the server 40. Such a record may comprise a count of repetitions and sets, the weight lifted by the user in the exercise activity, various timing data for the exercise activity, etc. The weight lifted may be detected by the local detector 20, for example as described hereinabove, or entered by the user into the user device 30 for the exercise activity. The timing data may designate the duration of the exercise activity or one or more phases of the exercise activity, such as the duration of a set, a repetition, an eccentric phase in a repetition, a concentric phase in a repetition, a pause between the eccentric and concentric phases, etc. As will be described in more detail below, the system may also be configured to provide real-time feedback to the user during the exercise activity, for example by presenting the number of repetitions and sets performed by the user, as well as an indication of the user movement during the exercise activity. The latter will allow the user to verify that the exercise activity is properly performed, for example in relation to a predefined movement pattern.

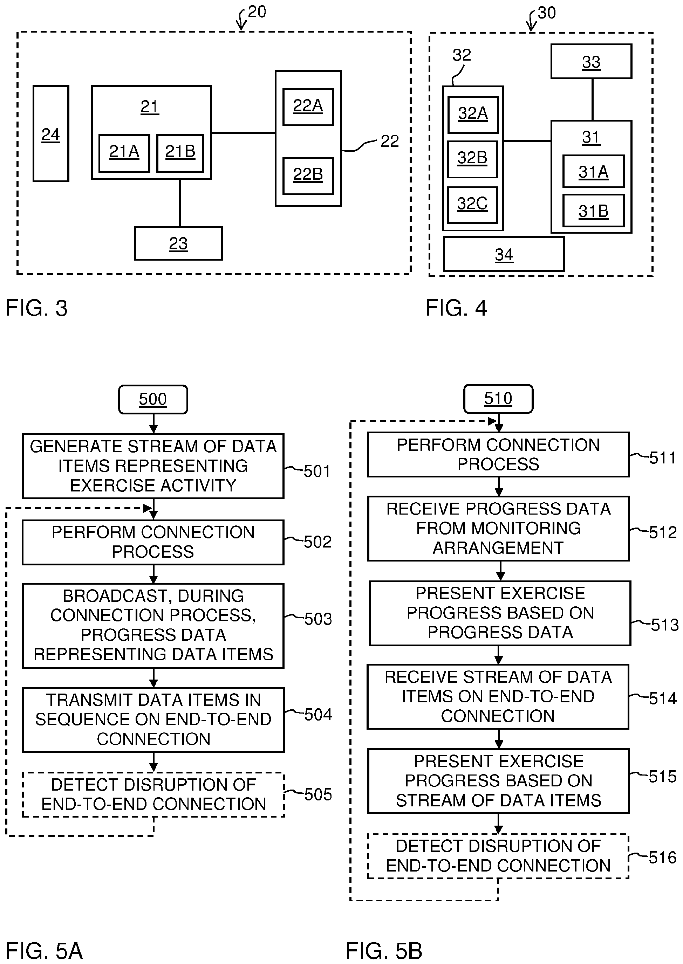

FIG. 3 is a block diagram of an example monitoring arrangement 20 ("local detector") in accordance with an embodiment. The components illustrated in FIG. 3 may be located in a housing or distributed. A control unit 21 ("control circuitry") is configured to implement logic for controlling the local detector 20. In the illustrated example, the control unit 21 comprises a combination of a processor 21A and a memory 21B. The memory 21B may store program instructions for execution by the processor 21A to implement the operation of the control unit 21. The program instructions may be supplied to the processor 21A on a computer-readable medium, which may be a tangible (non-transitory) product (for example, magnetic medium, optical disk, readonly memory, flash memory, etc.) or a propagating signal. The processor 21A may be a generic processor, for example a microprocessor, microcontroller, CPU, DSP (digital signal processor), GPU (graphics processing unit), etc., or a specialized processor, such as an ASIC (application specific integrated circuit) or an FPGA (field programmable gate array), or any combination thereof. The memory 21B may include volatile and/or non-volatile memory such as read only memory (ROM), random access memory (RAM) or flash memory.

The control unit 21 is connected to a communication device 22 and is operable to cause the communication device 22 to transmit a wireless signal. For brevity, the communication device 22 is denoted COMM in the following. In the example of FIG. 3, COMM 22 comprises a first module 23A, which is configured to perform the first mode of communication, and a second module 22B, which configured to perform the second mode of communication. The first and second modules 22A, 22B may be separate, dedicated circuits. The provision of separate circuits enables the use of different communication techniques and/or protocols in the first and second modes. In alternative embodiments, the functionality of the first and second modules 22A, 22B is implemented by a single circuit which is selectively switched to operate in the first mode or the second mode, or both. Provision of a single circuit will reduce cost and often also reduce complexity. In some embodiments, COMM 22 is configured for short-range communication in both the first mode and the second mode. In one implementation example, COMM 22 is configured for Bluetooth communication and comprises at least one Bluetooth transmitter. As used herein, a "Bluetooth transmitter" or "BT transmitter" refers to any wireless radio device capable of transmitting data in compliance with a Bluetooth SIG standard in any version. The term "transmitter" also encompasses transceivers. One specific type of BT transmitter is a BLE transmitter, which is configured to operate according to the Bluetooth Low Energy standard. Bluetooth Low Energy is also known as Bluetooth LE and Bluetooth Smart. As is known in the art, a BT transmitter may be switched between modes corresponding to the first and second modes, depending on the installed profiles. However, COMM 22 is not restricted to communication by Bluetooth but may use any wireless communication technique, including but not limited to Bluetooth, WiFi, ANT, Zigbee, Zwave, RFID, NFC, UWB, LR-WPAN, or ambient backscatter communication (ABC), either singly or in any combination. In other embodiments, a proprietary communication protocol may be applied in the first and/or second mode.

The local detector 20 in FIG. 3 further comprises the above-mentioned measurement device 23, which is connected by wire or wirelessly to the control unit 21, as well as a power source 24 for supplying power to the electrical components of the local detector 20. The power source 24 may include a battery, a fuel cell, an electrical connector for mains power, etc.

FIG. 4 is a block diagram of an example user device 30. A control unit 31 ("control circuitry") is configured to implement logic for controlling the user device 30. In the illustrated example, the control unit 31 comprises a combination of a processor 31A and a memory 31B. The memory 31B may store program instructions for execution by the processor 31A for implementing the operation of the control unit 31.

The program instructions may be supplied to the processor 31A on a computer-readable medium. The processor 31A may be a generic processor, for example a microprocessor, micro-controller, CPU, DSP (digital signal processor), GPU (graphics processing unit), etc., or a specialized processor, such as an ASIC (application specific integrated circuit) or an FPGA (field programmable gate array), or any combination thereof. The memory 31B may include volatile and/or non-volatile memory such as read only memory (ROM), random access memory (RAM) or flash memory.

The user device 30 further comprises a communication device 32 (denoted COMM in the following), which is at least partly configured in correspondence with COMM 22 of the local detector 20. Thus, COMM 32 is configured to implement the same communication technique and protocol as COMM 22. COMM 32 is operable in a first mode, in which it intercepts broadcasts. COMM 32 is also operable in a second mode, in which it establishes an end-to-end connection to the local detector 20 and receives a stream of data on the end-to-end connection. In the example of FIG. 4, the first mode is represented by module 32A, and the second mode is represented by module 32B. Like in FIG. 3, the functionality of the first and second modules 32A, 32B may be implemented by two separate, dedicated circuits or by a single circuit which is selectively operable in the first mode or the second mode, or both. COMM 32 is also operable to communicate with the server 40 (FIG. 2), either by one of the modules 32A, 32B or by a further module 32C. For example, the further module 32C may be configured for wireless or wired data transfer over a network, such as a WAN, LAN, PAN or any combination thereof.

In the illustrated example, the user device 30 further comprises a feedback device 33 for providing one or more of audible, visible or tactile feedback to the user. Such a feedback device 33 may comprise one or more of a speaker, a buzzer, a vibrator, a display, a touch screen, an indicator lamp, etc. In the following description, the feedback device 33 is assumed to comprise a display for visual presentation. The user device 30 also comprises a power source 34 for supplying power to the electrical components of the user device 30. The power source 34 may include a battery, a fuel cell, an electrical connector for mains power, etc.

The user device 30 may be a customized electronic device for use in the system of FIG. 2 or a generic device, such as a mobile phone, smartphone, tablet computer, etc. Such a generic device may be configured for use as a user device 30 in the system of FIG. 2 by a dedicated application program ("app") that is installed and executed by the control unit 31.

FIG. 5A is a flowchart of an example method 500 of operating the local detector 20 in accordance with an embodiment. The method 500 is performed by the control unit 21 in the local detector 20 (FIG. 3) while a user performs an exercise activity by the exercise device 10. In step 501, the measurement device 23 is operated to generate data items representing the exercise activity. As understood form the foregoing, the data items are generated as a stream or time sequence of data items. It should be understood that step 501 is performed as long as the user performs the exercise activity and thus also during subsequent steps of the method 500. In step 502, COMM 22 is operated to perform a connection process for establishment of an end-to-end connection to the user device 30. In some embodiments, described in further detail below, step 502 comprises operating COMM 22 to broadcast a connection request. Step 502 may be triggered by the user starting the exercise activity, as detected by the measurement device 23. In step 503, the communication device 23 is operated to start broadcasting "progress data" in the form of data packets. In other words, COMM 22 is operated in the first mode in step 503, and optionally also in step 502. The progress data represents the time-sequence of data items that are generated by the measurement device 23 in accordance with step 501 during the connection progress ("pre-connection data items"). It should be understood that the connection process may take some time to be completed, or even fail altogether. By step 503, the user device 30 is capable of receiving and presenting exercise feedback, given by the progress data, to the user even in the absence of an established end-to-end connection. To improve the ability of the user device 30 to intercept the progress data, COMM 22 may be operated to repeatedly broadcast the same data packet during a preconfigured time period and at a preconfigured interval, thereby providing a confined burst of the same data packet. It should be noted that the progress data need not be static but may be updated over time, in synchronization with the exercise activity. The content of the progress data may depend on the available bandwidth in the first mode. In some embodiments, all of the pre-connection data items are broadcast, with the respective data packet comprising an individual data item or a group of data items. In some embodiments, the progress data comprises a compressed representation of the pre-connection data items. In one example, every n:th data item is transmitted. In another example, when the data items represents a movement pattern, the progress data may comprise parameter values for a predefined parameterization of the movement pattern, thereby allowing the movement pattern to be reconstructed from the parameter values. In another example, the progress data comprises an indication of an achieved sub-target given by the pre-connection data items. As noted above, such a sub-target may be a repetition or a set that is performed by the user during the exercise activity. Thus, in step 503, the control unit 21 may process the pre-connection data items for detection of an achieved sub-target among a plurality of sub-targets for the exercise activity, and include an indication of the achieved sub-target in the progress data that is broadcast by COMM 22. In some embodiments, the control unit 21 is configured to maintain a count of the number of repetitions and/or sets performed by the user during the exercise activity and include the count in the progress data each time the count is updated.

In step 504, the end-to-end connection is established and COMM 22 is in the second mode. When the end-to-end connection is established, COMM 22 is operated to transmit the data items in sequence on the end-to-end connection to the user device 30. The data items are thereby transmitted in the order they are generated by the measurement device 23. The data items may be seen to be "streamed" on the end-to-end connection.

As indicated by dashed lines, the method 500 may comprise an optional step 505 of detecting a disruption of the end-to-end connection, causing the method 500 to reperform the connection process in accordance with step 502. The disruption may, for example, be signaled by COMM 22. The disruption may manifest itself as a lost or broken connection, or a significant drop in signal quality at COMM 22 or COMM 32. Depending on the configuration of COMM 22, 32, the signal quality may be given by signal strength, signal-to-noise ratio, transmission speed (if variable), lost data, number of retransmissions, or any other conventional measure.

The method 500 provides an improved technique of transferring exercise data from the exercise device 10 to the user device 30. The use of the end-to-end connection enables data items to be transferred in real time from the local detector 20 to the user device 30, which is thereby operable to provide exercise feedback in real time to the user. Depending on the data items and the generation rate at the measurement device 23, such real-time transfer of data items may enable quite rich and detailed exercise feedback, for example detailing the movement pattern performed by the user during the exercise activity. Another advantage of the end-to-end connection is that it may be implemented with various measures to ensure complete data transfer, such as transmission of acknowledgement data (ACK), detection of data loss, retransmission, encryption, etc.

The use of broadcasting during the connection process enables the user device 30 to present exercise feedback to the user also during the connection process. The method 500 will thereby reduce the impact of the duration of the connection process on the user experience, since exercise feedback may be presented on the user device 30 shortly after the user has started the exercise activity. Even if the broadcasting is implemented to have a relatively low bandwidth compared to the end-to-end connection, it is still possible to transfer sufficient exercise data (by the progress data) to provide exercise feedback that represents the progress of the exercise activity.

FIG. 5B is a flowchart of an example method 510 of operating the user device 30 in accordance with an embodiment. The method 510 is performed by the control unit 31. In step 511, COMM 32 is operated to perform a connection process for establishment of an end-to-end connection to the local detector 20. In some embodiments, the connection process is triggered by COMM 32 receiving a connection request, which is broadcast by the local detector 20. Thus, at step 511, COMM 32 may be operated in the first mode. In step 512, COMM 32 is operated, during the connection process, in the first mode to receive the above-mentioned progress data from the local detector 20. In step 513, the feedback device 33 is operated to present the progress of the exercise activity during the connection process, by use of the progress data. Step 514 is performed when the end-to-end connection is established. In step 514, COMM 32 is operated to receive a stream of data items on the end-to-end connection. In step 515, the feedback device 33 is operated to present the progress of the exercise activity after completion of the connection process, based on the data items. For example, the data items may be presented in real time as they arrive at COMM 32.

As indicated by dashed lines, the method 510 may comprise an optional step 516 of detecting a disruption of the end-to-end connection, causing the method 500 to reperform the connection process in accordance with step 511. The disruption may, for example, be signaled by COMM 32. Step 516 may be performed in correspondence with step 505 in method 500.

FIG. 6A is a sequence diagram of steps that may be performed in the system of FIG. 2 to enable transfer of exercise data. In step 601, the tag 1 of an exercise device is operated to broadcast the above-mentioned tag identifier, represented as MID1 in FIG. 6A. Step 601 may, for example, be triggered by a proximity sensor in the tag 1, or by the user pushing a button on the tag 1. In a non-limiting example, MID1 is a MAC address. In step 602, MID1 is intercepted by the user device 30, which is operated in the first mode. In step 603, the user device 30 transmits MID1 to the server 40, optionally together with an identifier of the user. Optionally, the user device 30 may prompt the user to enter further data to be transmitted to the server 40, for example to select one of a plurality of predefined exercise activities. In step 604, the server 40 receives MID1. In step 605, the server 40 determines an origin identifier MID2, based on MID1, for example by use of a look-up table, a database or the like. MID2 is associated with the exercise device 10 and is pre-stored in the local detector 20. In step 605, the server 40 may also derive metadata related to the exercise device 10 or the exercise activity, such as a name for the exercise device, a name for the exercise activity, a predefined movement pattern to be followed by the user, etc. In step 606, the server 40 transmits MID2, optionally together with the metadata, to the user device 30. In step 607, the user device 30 receives the transmitted data. In step 608, the user device 30 may activate the first mode, if not already activated, and display a message that identifies the exercise device, and optionally the exercise activity, and instructs the user to start the exercise activity. Steps 601-608 represents a "docking procedure", which prepares the user device 30 to receive exercise data from the local detector 20. In step 609, the local detector 20 detects that the exercise activity is started, based on sensor data from the measurement device 23. For example, start of an exercise activity may be inferred when an accelerometer detects a movement that exceeds a limit for a certain time period, or when a range sensor detects that a weight has been lifted for a predefined time period. In step 609, the local detector 20 may also switch from a low-power mode to an active mode. In step 610, the local detector 20 performs a connection process CP1 that involves broadcasting a connection request, which includes MID2. Step 610 corresponds to step 502 in FIG. 5A. In step 611, the user device 30 intercepts the connection request and extracts MID2. If MID2 from the user device 30 matches MID2 from the server 40, the user device 30 performs a connection process CP2. Step 611 corresponds to step 511 in FIG. 5B. In steps 612-613, the user device 30 and the local detector 20 exchange data and eventually establish the end-to-end connection, which causes the local detector 20 to perform step 504 in FIG. 5A and the user device 30 to perform step 514 in FIG. 5B.

FIG. 6A is a non-limiting example and many variants are conceivable. In one variant, the functionality of the server 40 is included in the user device 30. In another variant, the user manipulates the user device 30 in step 608 to perform the connection process CP2, for example by entering a command on the feedback device 33. The user device 30 thereby broadcasts a connection request, which includes MID2. The local detector 20 intercepts the connection request and extracts MID2. If MID2 from the user device 30 matches MID2 stored in the local detector 20, the local detector 20 performs the connection process CP1.

As understood from FIG. 5A, the local detector 20 is operable to either broadcast data (step 503) or stream data (step 504). FIG. 6C is a flow chart of an example broadcast procedure 620 that may be performed by the local detector 20 as part of step 503, and FIG. 6D is a flow chart of an example streaming procedure 630 that may be performed by the local detector 20 as part of step 504. As shown in FIG. 6B, the local detector 20 may be configured to switch from the broadcast procedure 620 to the streaming procedure 630, for example when the connection process is completed and the end-to-end connection has been established. As indicated by a dashed arrow, the local detector 20 may also be configured to switch back from the streaming procedure 630 to the broadcast procedure 620, for example upon detection of a disruption (step 505).

The procedures 620, 630 in FIGS 6C-6D are presented under the assumption that the data items generated by the measurement device 23 are momentary positions in one, two or three spatial dimensions. The positions may be relative or absolute positions. In the example of FIGS 1B-1C, the positions may correspond to the distance D. The procedures 620, 630 further presume that progress data is broadcast whenever a repetition is started and whenever a repetition is completed. In FIGS 6C-6D, a momentary position is designated POS, and progress data is designated PD.

The broadcast procedure 620 in FIG. 6C comprises a step 621 of obtaining a current position from the measurement device 23. In step 622, the current position is evaluated to determine if the exercise activity is completed. For example, the exercise activity may be deemed completed if a predefined number of consecutive positions correspond to a rest position for the exercise device 10. If the exercise activity is completed, a termination step 623 is performed. For example, the termination step 623 may comprise broadcasting a termination indicator (for receipt by the user device 30) and/or setting the local detector 20 in a low-power mode. Otherwise, the procedure proceeds to step 624, in which the current position is evaluated to detect the start of a new repetition, REPi. For example, REPi may be detected when the current position, optionally in combination with preceding positions, fulfils a dedicated start criterion, for example that the positions deviate significantly from the rest position of the exercise device, that the speed and/or acceleration has a characteristic pattern, etc. In the example of FIGS 1B-1C, REPi may be detected when the distance D has changed a predetermined amount from the rest distance and/or when the distance D starts to increase after having decreased. If REPi is detected, the procedure proceeds to step 625, in which a repetition counter is updated (incremented), and step 626, in which progress data is broadcast. The progress data includes the current value of the repetition counter ("current rep count"). After step 626, the procedure evaluates if the end-to-end connection has been established in step 629. If so, the procedure 620 is terminated and the procedure 630 is started. Otherwise, the procedure 620 returns to step 601. If REPi is not detected in step 624, the procedure proceeds to step 627, in which the current position is evaluated for detection of a completed repetition, REPc. REPc may be detected by analogy with REPi. If REPc is detected, the procedure proceeds to step 628, in which progress data is broadcast. The progress data may include a "rep complete" indicator, the current rep count, as well as timing data for one or more phases during the repetition. After step 628, the procedure proceeds to step 629. If REPc is not detected in step 627, the procedure returns to step 621.

In some embodiments, all broadcasts of progress data include a connection request, to increase the probability that the connection request is intercepted by the user device 30. Further, in some embodiments, the first connection request is broadcast upon detection of the first REPi, i.e. when the user starts the first repetition of the exercise activity. This means that the broadcasting of progress data (step 503) is part of the connection process for establishing the end-to-end connection (step 502). In other words, step 502 is included in step 503. It may also be noted that all broadcasts performed by the local detector 20 includes the origin identifier, MID2.

The streaming procedure of FIG. 6D comprises steps 631-633, which correspond to steps 621-623 in the broadcast procedure 620. If completion of the exercise activity is not detected in step 632, the procedure may proceed to step 636, in which the current position is transmitted on the end-to-end connection. In subsequent step 637, the procedure may evaluate if the end-to-end connection is disrupted. Thus, step 637 corresponds to step 505. If a disruption is detected, the procedure 630 may be terminated and the procedure 620 started. Thus, as long as the exercise activity is not completed or a disruption occurs, the procedure 630 repeatedly obtains and transmits current positions on the end-to-end connection.

The procedure 630 may comprise a step 634 of evaluating the current position, optionally in combination with previous positions, to detect the start of a new repetition, REPi. If REPi is detected, the procedure proceeds to step 635, in which the repetition counter is updated (incremented). Steps 634-635 may correspond to steps 624-625. By steps 634-635, the repetition counter will represent the actual number of repetitions performed by the user during the exercise activity, if and when step 637 switches from the streaming procedure 630 back the broadcast procedure 620. As indicated by the dashed lines, steps 634-635 are optional. In an alternative, the user device 30 may instead be configured to implement steps 634-635 to keep an updated count of the number of repetitions based on the stream of positions that are received on the end-to-end connection.

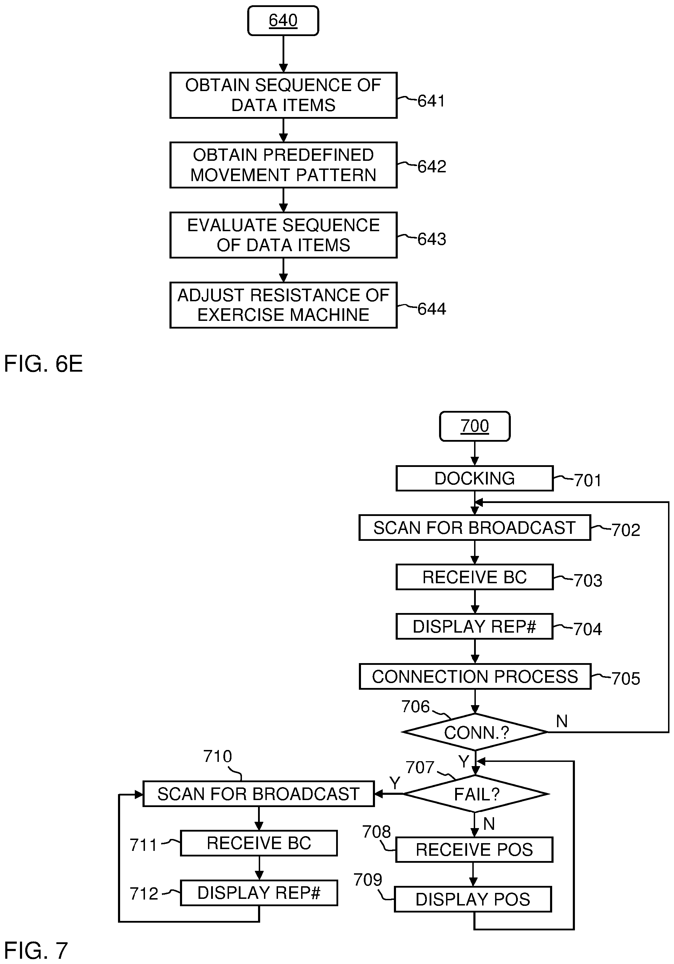

FIG. 6E is a flow chart of a control procedure 640 that may be performed in the system of FIG. 2. The control procedure 640 automatically adjusts the resistance of an exercise machine based on a sequence of data items generated by the measurement device 23. As used herein, the resistance refers to the inertia experienced by the user during the exercise activity and is thus equivalent to the weight that is lifted by the user. Reverting to FIG. 2, the exercise machine 10 comprises a resistance controller 19, which is operable to adjust the resistance of the exercise machine 10, in steps or continuously. For example, the resistance controller 19 may apply a variable force on a mechanical linkage in the exercise machine 10 to change the resistance experienced by the user. In the example of FIG. 2, the local detector 20 is connected to provide an adjustment signal (dashed arrow) to the resistance controller 19 to adjust the resistance. In FIG. 6E, the procedure 640 comprises a step 641 of obtaining a sequence of data items, for example momentary positions that define a movement pattern performed by the user ("current pattern"). In step 642, a predefined movement pattern ("predefined pattern") for the exercise activity is obtained. In step 643, the sequence of data items is evaluated in relation to the predefined pattern, for example to quantify the similarity or difference between a current pattern and the predefined pattern. In step 644, the resistance of the exercise machine is adjusted based on the evaluation in step 643. For example, if a significant difference is detected in step 643 and attributed to the resistance being too low, the resistance may be increased. Conversely, if the significant difference is attributed to the resistance being too high, the resistance may be decreased. In another example, if a high degree of similarity of detected in step 643, the resistance may be increased. By the automatic adjustment, the procedure 640 may optimize the resistance to the capabilities of the user and, for example, enable the user to perform the exercise activity to failure with good form.

In some embodiments, the procedure 640 is performed by the user device 30 based on the stream of data items received from the local detector 20. The predefined pattern may be pre-stored in memory of the user device 30 or may be received from the server 40. In the example of FIG. 2, the user device 30 may transmit an adjustment command for the local detector 20 on the end-to-end connection, causing the local detector 20 to operate the resistance controller 19 accordingly. Alternatively, the user device 30 may be otherwise operable to transmit an adjustment signal to the resistance controller 19.

In some embodiments, the procedure 640 is performed by the local detector 20, based on the sequence of data items generated by the measurement device 32. The local detector 20 may obtain the predefined pattern from the user device 30 on the end-to-end connection.

In some embodiments, the procedure 640 is performed by the server 40, which may receive the stream of data items from the user device 30. If the server 40 has a direct communication path to the resistance controller 19, the server may transmit an adjustment signal to the resistance controller 19. Alternatively, the server 40 may transmit an adjustment command to the local detector 20 via the user device 30.

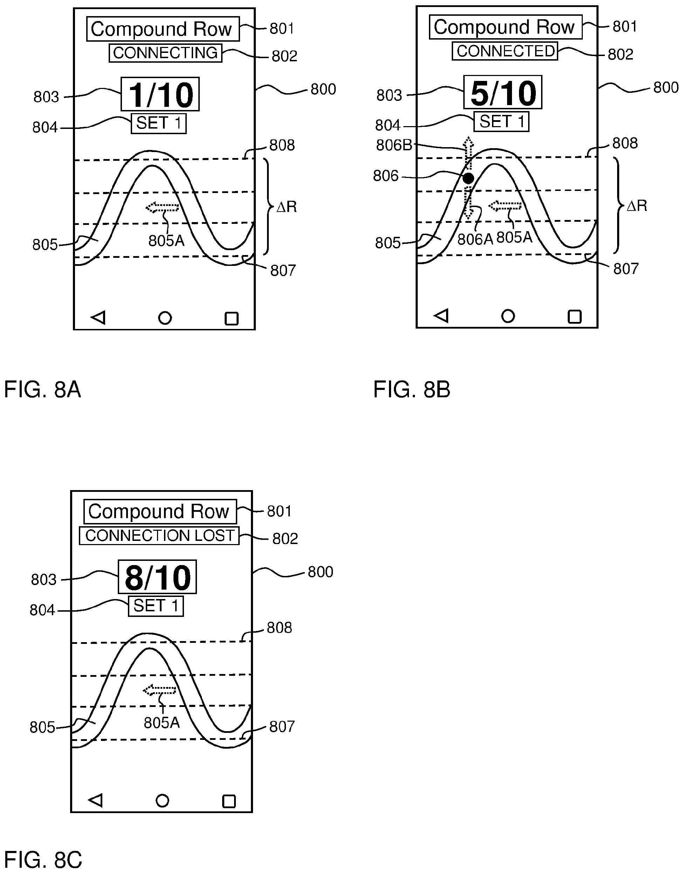

FIG. 7 is a flow chart of a feedback procedure 700 performed by the user device 30 while the local detector 20 is operated in accordance with FIGS 6A-6D. The procedure 700 will be described with further reference to FIGS 8A-8C, which show examples of the content displayed on the user device 30. In step 701, the user device performs the above-mentioned docking operation. In the example of FIG. 6A, the docking step 701 corresponds to steps 602, 603 and 607. In step 702, the user device is configured to scan for broadcasts, by operating COMM 32 in broadcast mode. Step 702 corresponds to step 608 in FIG. 6A. In step 703, the user device intercepts a broadcast packet (BC) from the local detector 20 and extracts the current rep count. In step 704, the user device displays a number (REP#) corresponding to the current rep count. If BC comprises a connection request, step 705 performs the connection process. If the end-to-end connection is not established at step 706, the procedure returns to step 702. Steps 702-705 are thereby repeatedly performed until the end-to-end connection is established. In this process, step 704 is performed to update the displayed number (REP#) whenever an updated current rep count is extracted from BC in step 703. Should the connection process fail during the repetition of steps 702-705, the connection process will be continued in step 705 upon receipt of a connection request in step 703. It is realized that the repetition of steps 702-705 corresponds to steps 511-513 in FIG. 5B.

FIG. 8A shows an example of an image 800 which is displayed on the user device during the repetition of steps 702-705. In the illustrated example, the image 800 comprises a field 801 designating the type of exercise activity ("compound row"), and a field 802 designating the current status of the end-to-end connection. In FIG. 8A, the status is "connecting" since the connection process is in progress. Field 803 designates the current repetition (REP#). In the illustrated example, the total number of repetitions to be performed is also displayed in field 803. Field 804 designates the current set. A predefined movement trajectory 805 for a repetition is graphically depicted as well. In the illustrated example, the trajectory 805 is a sinusoid representing a concentric movement of the user, for example corresponding to weights being lifted (FIG. 1C) followed by an eccentric movement, for example corresponding to weights being lowered (FIG. 1B). The trajectory 805 may have any shape and be a generic pattern or customized for the user. The trajectory 805 is displayed in relation to a vertical dimension representing position (for example distance D, FIGS 1B-1C) and a horizontal dimension representing time. As indicated by a dashed arrow 805A, the trajectory 805 is displaced (swept) from right to left at a predefined speed. The combination of the trajectory 805 and the speed defines a predefined movement pattern, which is to be followed by the user when performing the exercise activity. It has been found that this presentation of the predefined pattern does indeed assist the user in performing the exercise activity, by allowing the user to synchronize movements with the pattern. In the illustrated example, horizontal lines 807, 808 are displayed to further guide the user. The lines 807, 808 represent the extreme positions during a repetition. In FIG. 8A, the distance ΔR between the lines 807, 808 corresponds to the range of motion (RoM) for the exercise activity. As used herein, "range of motion" refers to the range of positions generated by the measurement device 23 during a repetition.

It is realized that it may be relevant to present the display image 800, as well as establish the end-to-end connection, as soon as possible when the user starts the exercise activity. This may be achieved by the local detector 20 broadcasting a connection request directly upon detection of the start together with a current rep count set to one (1), for example in accordance with steps 624-626 in FIG. 6C.

Reverting to FIG. 7, the procedure 700 proceeds to step 707 when the end-to end connection is established. In step 707, the user device checks if the end-to-end connection has been disrupted. As long as the end-on-end connection is active, the user device repeatedly performs steps 708-709 to receive a current position on the end-to-end connection and present the current position on the display (cf. steps 514-515 in FIG. 5B).

FIG. 8B shows the image 800 as displayed during the repetition of steps 708-709. Compared to FIG. 8A, field 802 has been updated to indicate status "connected" since the end-to-end connection is active, and field 803 has been updated to indicate the current repetition (5 of 10). The current repetition may be determined by the user device processing the incoming stream of positions for detection of REPi (cf. step 624 in FIG. 6C). Further, a dot 806 is displayed to indicate the current position in relation to the movement pattern. The current position is given by step 709 in FIG. 7. As indicated by arrow 805A, the trajectory 805 is still displaced in accordance with the predefined speed. The vertical position of the dot 806 is adjusted in the vertical direction, as indicated by arrows 806A, 806B, in accordance with the current position. The dot 806 thereby provides real-time feedback to the user about the user's actual performance of the exercise activity in relation to the predefined pattern. The user is also given the ability to synchronize the dot 806 with the pattern, by adjusting the user movements so as to keep the dot 806 aligned with the trajectory 805.

In some embodiments, the user device 30 is configured to provide, via the feedback device 33, specific instructional feedback related to the user's performance of the exercise activity. The user device 30 may generate the instructional feedback by evaluating one or more momentary positions in the stream of positions in relation to the predefined pattern. The momentary position(s) may be evaluated in the time domain and/or the spatial domain. The instructional feedback may include developmental feedback to correct the user's performance, for example the pace of the user or the range of motion. Additionally or alternatively, the instructional feedback may include motivational feedback when the user succeeds to align the exercise activity with the predefined pattern. The instructional feedback may be given in real time, in-between sets, after completion of the exercise activity, or in any combination thereof.

Reverting to FIG. 7, the procedure 700 proceeds to step 710 if the user device detects a disruption in step 707. Steps 710-712 correspond to steps 702-704 and are performed repeatedly. Thereby, the user device is operated to scan for broadcasts (step 710), intercept a broadcast packet (BC) from the local detector 20 and extract the current rep count (step 711), and display a number (REP#) corresponding to the current rep count (step 712). Although not shown in FIG. 7, the user device may also be operated to re-establish the end-to-end connection in correspondence with step 705.

FIG. 8C shows the image 800 as displayed during the repetition of steps 710-712. Compared to FIG. 8B, field 802 has been updated to indicate status "connection lost", and field 803 has been updated to indicate the current repetition (8 of 10). Further, the dot 806 is no longer displayed, in the absence of a current position. As indicated by arrow 805A, the trajectory 805 is still displaced in accordance with the predefined speed.

The image 800 shown in FIG. 8B requires RoM to be known in order for the dot 806 to be correctly positioned within ΔR. In certain types of exercise machines, RoM may predefined and given by mechanical constraints. However, in other types of exercise machines, such as cable machines, which enable the user to perform different exercise activities in different ways, RoM may differ significantly between exercise activities and/or users, and also between different training sessions by the same user. The same applies to free weights. In some embodiments, to address this problem, the user device 30 may be configured to perform an automatic calibration of incoming positions to correctly locate the dot 806 within ΔR on the display.

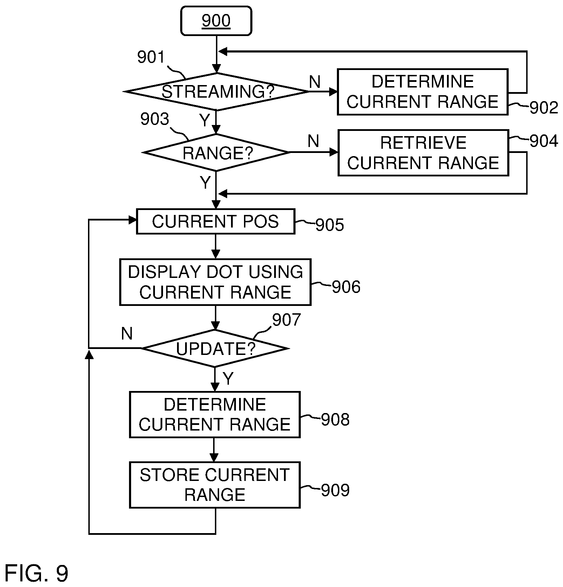

FIG. 9 is a flowchart of a procedure 900 that includes such an automatic calibration. In step 901, if the end-to-end connection is not established, the procedure proceeds to step 902, in which a current RoM is determined based on progress data obtained from broadcasts (cf. BC in FIG. 7) that are intercepted by the user device. Step 902 presumes that the local detector 20 is configured to include, in one or more of its broadcasts of progress data, at least a minimum position and a maximum position attained during a repetition, thereby allowing the user device to determine the current RoM as a function of the difference between the minimum and maximum positions. Steps 901-902 may be repeated until the end-to-end connection is established, whereupon the procedure proceeds to step 903. In step 903, if the user device has failed to determine a current range, the procedure proceeds to step 904, in which a current range is retrieved from internal memory or from the server 40 (FIG. 2). Thereby, at subsequent step 905, the user device has obtained a current RoM. In step 905, the user device receives a current position on the end-to-end connection. In step 906, the user device positions the dot 806 within ΔR by use of the current RoM. For example, the user device may convert the current position, by use of the current RoM, into a target location on the display, and operate the feedback device 33 to display the dot 806 at the target location. Step 906 may be seen to match the target location in relation to ΔR to the current position in relation to the current RoM. For example, the ratio between ΔR and the current RoM may be applied as a scaling factor to the current position. In step 907, the user device determines if the current RoM should be updated. For example, an update may be triggered when a predefined number of repetitions has been started or completed, or based on a variability among positions obtained by step 905. If no update is triggered, the procedure returns to step 905. If an updated is triggered in step 907, the procedure proceeds to step 908, in which a current RoM is determined based on a sequence of positions obtained by the repeated execution of step 905. For example, step 907 may comprise detecting a minimum position and a maximum position in the sequence of positions, and determining the current RoM as a function of the difference between the minimum and maximum positions. In step 909, the current RoM may be stored, for example in internal memory or at the server 40. The thus-stored current RoM may be retrieved in a forthcoming execution of step 904. The procedure then returns to step 905. It is realized that step 902 and step 908 provide an automatic calibration during broadcast and streaming, respectively.

While the subject of the present disclosure has been described in connection with what is presently considered to be the most practical embodiments, it is to be understood that the subject of the present disclosure is not to be limited to the disclosed embodiments, but on the contrary, is intended to cover various modifications and equivalent arrangements included within the spirit and the scope of the appended claims.

Further, while operations are depicted in the drawings in a particular order, this should not be understood as requiring that such operations be performed in the particular order shown or in sequential order, or that all illustrated operations be performed, to achieve desirable results.

In the following, clauses are recited to summarize some aspects and embodiments as disclosed in the foregoing.

C1. A monitoring arrangement for installation in an exercise device (10), said monitoring arrangement comprising a measurement device (23) and a communication device (22) for wireless communication and being configured to: generate, by the measurement device (23), data items representing an exercise activity performed by a user of the exercise device (10); perform, by the communication device (22), a connection process for establishment of an end-to-end connection to a user device (30); operate the communication device (22), during said connection process, to broadcast progress data that represents the data items that are generated during said connection process; and operate the communication device (22), after establishment of the end-to-end connection, to sequentially transmit on the end-to-end connection the data items that are generated after said establishment of the end-to-end connection.

C2. The monitoring arrangement of C1, wherein the data items comprise momentary positions of the exercise device (10) or a moveable element (11) of the exercise device (10).

C3. The monitoring arrangement of C1 or C2, wherein the data items represent a movement pattern.

C4. The monitoring arrangement of any preceding clause, which is further configured to, during said connection process, process the data items for detection that a respective sub-target among a plurality of sub-targets for the exercise activity is achieved, and broadcast the progress data when the respective sub-target is achieved, wherein the progress data comprises an indication of the respective sub-target.

C5. The monitoring arrangement of C4, wherein the respective sub-target corresponds to start or completion of an exercise movement performed by the user as part of the exercise activity.

C6. The monitoring arrangement of C4 or C5, which is configured to repeatedly process the data items and update a count of sub-targets achieved by the user during the exercise activity, and wherein the monitoring arrangement is configured to, during said connection process, operate the communication device (22) to, whenever the count is updated, include the count in the progress data and broadcast the progress data.

C7. The monitoring arrangement of any preceding clause, wherein the communication device (22) is configured for wireless short-range communication.

C8. The monitoring arrangement of any preceding clause, wherein the communication device (22) comprises one or more Bluetooth transceivers and is configured to operate the one or more Bluetooth transceivers to establish the end-to-end connection and to broadcast the progress data, respectively.

C9. The monitoring arrangement of any preceding clause, which is further configured to detect, by the measurement device (23), a start of the exercise activity, and, upon detecting the start, perform said connection process.

C10. The monitoring arrangement of any preceding clause, which is configured to perform said connection process by broadcasting a connection request for establishment of the end-to-end connection.

C 11. The monitoring arrangement of C10, which is configured to repeatedly broadcast the connection request during the connection process.

C12. The monitoring arrangement of C10 or C 11, which is configured to broadcast the progress data in combination with the connection request.

C13. The monitoring arrangement of any preceding clause, which is configured to operate the communication device (22) to transmit the data items in real time to the user device (30) on the end-to-end connection.

C14. The monitoring arrangement of any preceding clause, which is further configured to detect a disruption of the end-to-end connection and, upon detecting the disruption, operate the communication device (22) to broadcast further progress data that represents a time-sequence of said data items generated after the disruption.

C15. The monitoring arrangement of C3, which is further configured to perform an evaluation of the movement pattern in relation to a predefined movement pattern; determine, based on the evaluation, a modification of the exercise activity; and apply the modification by selectively causing a resistance controller (19) of the exercise device (10) to adjust a resistance of the exercise activity while the user performs the exercise activity.

C16. The monitoring arrangement of any preceding clause, wherein the measurement device (23) is configured to generate each of the data items to represent a momentary vertical position of at least one of a plurality of stacked weights in the exercise machine (10).

C17. An exercise device configured to be operated by a user to perform an exercise activity, said exercise device comprising the monitoring arrangement of any preceding clause.

C18. A computer-implemented method of operating a monitoring arrangement, said monitoring arrangement being configured for installation in an exercise device and comprising a measurement device and a communication device, said method comprising: generating (501), by the measurement device, data items representing an exercise activity performed by a user of the exercise device; performing (502), by the communication device, a connection process for establishment of an end-to-end connection to a user device; operating (503) the communication device, during said connection process, to broadcast progress data that represents the data items that are generated during said connection process; and operating (504) the communication device (22), after establishment of the end-to-end connection, to sequentially transmit on the end-to-end connection the data items that are generated after said establishment of the end-to-end connection.

C19. An electronic device comprising a communication device (32) and a feedback device (33), said electronic device being configured to: perform, by the communication device (32), a connection process for establishment of an end-to-end connection to a monitoring arrangement (20) of an exercise device (10); operate, during the connection process, the communication device (32) in a broadcast mode to receive progress data from the monitoring arrangement (20), said progress data representing an exercise activity performed by a user of the exercise device (10) during the connection process; operate the feedback device (33) to present progress of the exercise activity based on the progress data; operate the communication device (32), upon said establishment of the end-to-end connection, to receive on the end-to-end connection a sequence of data items, wherein the sequence of data items represents the exercise activity performed by a user of the exercise device (10) after said establishment of the end-to-end connection; and operate the feedback device (33) to present the progress of the exercise activity based on the data items.

C20. The electronic device of C19, which is configured to perform the connection process upon receipt, by the communication device (32) operated in the broadcast mode, of a connection request from the monitoring arrangement.

C21. The electronic device of C20, which is configured to extract from the connection request a start indicator, which is indicative of a start of the exercise activity, and operate the feedback device (33) to present the start of the exercise activity.

C22. The electronic device of any one of C19-C21, wherein the data items comprises momentary positions of the exercise device (10) or a moveable element (11) of the exercise device (10).

C23. The electronic device of C22, wherein the feedback device (33) comprises a display, and wherein the electronic device is configured to operate the feedback device (33) to present the sequence of data items on the display by displaying a momentary position for a respective data item as a dot (806) in relation to a predefined movement pattern (807) for the exercise activity.

C24. The electronic device of C23, which is configured to present the respective data item, on the display, in real time as the respective data item is received on the end-to-end connection.

C25. The electronic device of C23 or C24, which is configured to process the data items and/or the progress data to determine a range of motion of the exercise device (10) or the moveable element (11) during the exercise activity; convert the momentary position, by use of the range of motion, into a target location on the display (33); and operate the feedback device (33) to present the dot (806) at said target location.

C26. The electronic device of C25, which is configured to operate the feedback device (33) to present the predefined movement pattern (807) on the display with a vertical extent (ΔR) that corresponds to the range of motion, and wherein electronic device is configured to convert the momentary position so that the target location, in relation to the vertical extent (ΔR), matches the momentary position in relation to the range of motion.

C27. The electronic device of any one of C23-C26, which is configured to operate the feedback device (33) to present the predefined movement pattern (807) on the display upon receipt of a start indication.

C28. The electronic device of any one of C23-C27, which is configured to evaluate the momentary position in relation to the predefined movement pattern (807) for determination of instructional feedback, and operate the feedback device (33) to present the instructional feedback to the user.

C29. The electronic device of any one of C19-C28, wherein the progress data comprises a count of achieved sub-targets during the exercise activity, and wherein the electronic device is configured to operate the feedback device (33) to present the count.

C30. The electronic device of any one of C19-C29, which is further configured to detect a disruption of the end-to-end connection; operate, upon detecting the disruption, the communication device (32) in a broadcast mode to receive further progress data that represents a time-sequence of said data items generated after the disruption; and operate the feedback device (33) to present the progress of the exercise activity after said disruption based on the further progress data.

C31. The electronic device of any one of C 19-C30, wherein the sequence of data items represents a movement pattern, and wherein the electronic device is configured to perform an evaluation of the movement pattern in relation to a predefined movement pattern; determine, based on the evaluation, a modification of the exercise activity; and apply the modification by selectively causing a resistance controller (19) of the exercise device (10) to adjust a resistance of the exercise activity while the user performs the exercise activity.

C32. A computer-implemented method of operating an electronic device, which comprises a communication device and feedback device, said method comprising: performing (511), by the communication device, a connection process for establishment of an end-to-end connection to a monitoring arrangement of an exercise device; operating (512), during the connection process, the communication device in a broadcast mode to receive progress data from the monitoring arrangement, said progress data representing an exercise activity performed by a user of the exercise device during the connection process; operating (513) the feedback device to present progress of the exercise activity based on the progress data; operating (514) the communication device, upon said establishment of the end-to-end connection, to receive on the end-to-end connection a sequence of data items, wherein the sequence of data items represents the exercise activity performed by a user of the exercise device (10) after said establishment of the end-to-end connection; and operating (515) the feedback device to present the progress of the exercise activity based on the data items.

C33. A computer-readable medium comprising computer instructions which, when executed by processing circuitry (21A; 31A), cause the processing circuitry (21A; 31A) to perform the method of C18 or C32.

## Claims

1. A monitoring arrangement for installation in an exercise device (10), said monitoring arrangement comprising a measurement device (23) and a communication device (22) for wireless communication and being configured to:
generate, by the measurement device (23), data items representing an exercise activity performed by a user of the exercise device (10),
perform, by the communication device (22), a connection process for establishment of an end-to-end connection to a user device (30),
operate the communication device (22), during said connection process, to broadcast progress data that represents the data items that are generated during said connection process, and
operate the communication device (22), after establishment of the end-to-end connection, to sequentially transmit on the end-to-end connection the data items that are generated after said establishment of the end-to-end connection.

2. The monitoring arrangement of claim 1, wherein the data items comprise momentary positions of the exercise device (10) or a moveable element (11) of the exercise device (10).

3. The monitoring arrangement of claim 1 or 2, which is further configured to, during said connection process, process the data items for detection that a respective sub-target among a plurality of sub-targets for the exercise activity is achieved, and broadcast the progress data when the respective sub-target is achieved, wherein the progress data comprises an indication of the respective sub-target.

4. The monitoring arrangement of claim 3, wherein the respective sub-target corresponds to start or completion of an exercise movement performed by the user as part of the exercise activity.

5. The monitoring arrangement of claim 3 or 4, which is configured to repeatedly process the data items and update a count of sub-targets achieved by the user during the exercise activity, and wherein the monitoring arrangement is configured to, during said connection process, operate the communication device (22) to, whenever the count is updated, include the count in the progress data and broadcast the progress data.

6. The monitoring arrangement of any preceding claim, which is further configured to detect, by the measurement device (23), a start of the exercise activity, and, upon detecting the start, perform said connection process.

7. The monitoring arrangement of any preceding claim, which is configured to perform said connection process by broadcasting a connection request for establishment of the end-to-end connection.

8. The monitoring arrangement of claim 7, which is configured to broadcast the progress data in combination with the connection request.

9. The monitoring arrangement of any preceding claim, which is configured to operate the communication device (22) to transmit the data items in real time to the user device (30) on the end-to-end connection.

10. The monitoring arrangement of any preceding claim, wherein the data items represent a movement pattern, and wherein the monitoring arrangement is further configured to perform an evaluation of the movement pattern in relation to a predefined movement pattern; determine, based on the evaluation, a modification of the exercise activity; and apply the modification by selectively causing a resistance controller (19) of the exercise device (10) to adjust a resistance of the exercise activity while the user performs the exercise activity.

11. An electronic device comprising a communication device (32) and a feedback device (33), said electronic device being configured to:
perform, by the communication device (32), a connection process for establishment of an end-to-end connection to a monitoring arrangement (20) of an exercise device (10),
operate, during the connection process, the communication device (32) in a broadcast mode to receive progress data from the monitoring arrangement (20), said progress data representing an exercise activity performed by a user of the exercise device (10) during the connection process,
operate the feedback device (33) to present progress of the exercise activity based on the progress data,
operate the communication device (32), upon said establishment of the end-to-end connection, to receive on the end-to-end connection a sequence of data items, wherein the sequence of data items represents the exercise activity performed by a user of the exercise device (10) after said establishment of the end-to-end connection, and
operate the feedback device (33) to present the progress of the exercise activity based on the data items.

12. The electronic device of claim 11, which is configured to perform the connection process upon receipt, by the communication device (32) operated in the broadcast mode, of a connection request from the monitoring arrangement, and extract from the connection request a start indicator, which is indicative of a start of the exercise activity, and operate the feedback device (33) to present the start of the exercise activity.

13. The electronic device of claim 11 or 12, wherein the data items comprises momentary positions of the exercise device (10) or a moveable element (11) of the exercise device (10), wherein the feedback device (33) comprises a display, wherein the electronic device is configured to operate the feedback device (33) to present the sequence of data items on the display by displaying a momentary position for a respective data item as a dot (806) in relation to a predefined movement pattern (807) for the exercise activity.

14. The electronic device of claim 13, wherein the electronic device is further configured to process the data items and/or the progress data to determine a range of motion of the exercise device (10) or the moveable element (11) during the exercise activity; convert the momentary position, by use of the range of motion, into a target location on the display (33); and operate the feedback device (33) to present the dot (806) at said target location.

15. The electronic device of any one of claims 11-14, wherein the sequence of data items represents a movement pattern, and wherein the electronic device is configured to perform an evaluation of the movement pattern in relation to a predefined movement pattern; determine, based on the evaluation, a modification of the exercise activity; and apply the modification by selectively causing a resistance controller (19) of the exercise device (10) to adjust a resistance of the exercise activity while the user performs the exercise activity.
